# EUROPEAN PATENT SPECIFICATION

(11) **EP 2 012 768 B1**
(45) Date of publication and mention of the grant of the patent: **26.11.2014**
(21) Application number: 07755339.4
(22) Date of filing: 13.04.2007
(51) Int. Cl.: A61K 31/19, A61K 31/215, A61K 9/70, A61P 1/02, A61K 36/888, A61K 31/573, A61K 9/00, A61K 31/58, A61K 31/704, A61K 36/484

(54) **DIMPLE, ERODIBLE ORAL ADHERING TROCHE**
Konkave, erodierbare, oral-haftende Pastille
PASTILLE CONCAVE, ÉRODABLE ET ADHÉSIVE POUR L'APPLICATION ORALE

(30) Priority: 13.04.2006 US 792121 P
(43) Date of publication of application: 14.01.2009
(73) Proprietor: Orahealth Corporation, Bellevue WA 98005 (US)
(72) Inventor: Haley, Jeffrey, T., Bellevue, WA 98005-4211 (US)
(74) Representative: Fleuchaus, Michael A.
(86) International application number: PCT/US2007/009032
(87) International publication number: WO 2007/120768

(56) References cited:
- WO-A-2004/043351
- KR-A- 20030 054 221
- KR-A- 20050 055 858
- US-A- 4 406 882
- US-A- 6 149 615
- US-A1- 2004 156 930
- US-B1- 6 197 331
- HALEY J T ET AL: "Studies of licorice extract in an oral patch on minor aphthous ulcers" ORAL SURGERY, ORAL MEDICINE, ORAL PATHOLOGY, ORAL RADIOLOGY ANDENDODONTICS, MOSBY-YEAR BOOK, ST. LOUIS, MO, US, vol. 99, no. 4, 1 April 2005 (2005-04-01), page 429, XP004787498 ISSN: 1079-2104
- FINNEY R.S. ET AL.: 'The pharmacological properties of glycyrrhetinic acid: a new anti-inflammatory drug' J. PHARM. PHARMACOL. vol. 10, no. 11, 1958, pages 687 - 695, XP008130693
- KRAUS S.D.: 'Glycyrrhetinic acid-a triterpene with antiestrogenic and anti-inflammatory activity' J. PHARM. PHARMACOL. vol. 12, 1960, pages 300 - 306, XP008130694

## Description

### BACKGROUND

To deliver a medication in the mouth over time for treatment of health problems in the mouth or throat, oral patches have been developed.

As used herein, the word "patch" does not include preparations that move about the mouth rather than adhering in one place, such as cough drops or throat lozenges, and therefore do not maintain a high concentration of released medication in the desired spot. Nor does it include preparations that do not hold together as a single item when held in the mouth such as preparations of powder, liquid, paste, viscous liquid gel, or a tablet or troche that crumbles into a powder or paste when chewed or placed in saliva. Conversely, it does include an adherent preparation formed of a hydrocolloid that holds together as a single item when held in the mouth, such as the adherent, soluble oral patch disclosed by the same inventor in US patent application serial number 10/287,843 filed November 5, 2002 (WO 2004/043351) and US Patent application US 2004/156930.

The most significant differences between an oral patch as used herein and other forms of medicinal preparations is that an oral patch is designed to release medication into the mouth over a relatively long period of time, such as 30 minutes or more, and be adherent to stay in one place so that the medication can reach high concentrations along side the patch, and remain in the mouth as a single item that will not spread to be in a plurality of locations in the mouth at one time.

### SUMMARY OF THE INVENTION

Through trials, the inventor has discovered that, directly placing glycyrrhetinic acid ("GTA") which may be extracted from licorice root (glycyrrhiza) in an adhesive oral patch for treatment of ordinary mouth ulcers (also called denture sores, canker sores and aphthous ulcers) is effective for relieving pain and speeding of healing. Thus the present invention provides a dimple, erodible, oral adhearing troche according to the claims. Preferred embodiments are described in the dependent claims.

When the oral adhering troche is adhered to teeth, orthodontic braces or a canker sore for longer than 15 minutes, the canker sore pain is significantly reduced and there is no numbing of surrounding tissues. The pain relief continues while eating long enough to complete a meal with reduced pain. The patch with e.g. GTA also speeds healing.

GTA base may be used in the oral patch. However, for higher levels of absorption into local tissues and therefore greater effectiveness for the level of drug used (to minimize risks of side effects) it is preferable to use a salt of glycyrrhetinic licorice extract that is water soluble at human mouth temperatures (Soluble Glycyrrhetinic Extract, "SGE"). SGE comprises a group of chemical salts of glycyrrhetinic acid that are soluble in water at human mouth temperatures, including potassium salt of glycyrrhetinic acid and other alkali metal salts of glycyrrhetinic acid.

In one aspect, the invention is a method for treating canker sores by providing patches which, when exposed to saliva in a human mouth, release e.g. GTA over more than 30 minutes, and instructing people to hold the patches in their mouths on or near the canker sore for at least 2 or more hours per day. The patch may include a binder ingredient to hold and release the medication.

The binder ingredients may be a combination of gums that dissolve in saliva, such as gum Arabic (acacia gum), carrageenan, xanthan gum, konjac gum, agar, or locust bean gum and non-dissolving food fibers. If the binders are xanthan gum, konjac gum, and cellulose fiber, effective dry weight formulations have between 1% and 10% SGE, such as potassium salt of GTA, between 20% and 55% food grade gelatin, and between 20% and 75% other binders. Another effective formula has 2-4% SGE with about 5-7% benzocaine and 50 - 93% gelatin, with acacia gum added on a side intended to be more adherent.

### BRIEF DESCRIPTION OF THE DRAWING

Figure 1a shows a side view of an oral patch that completely dissolves (erodes).
Figure 1b shows a top view of the same oral patch.
Figure 2 shows a layered oral patch covering a canker sore.
Figure 3 shows a domed oral patch with a dimple made by pressing powders.

### DETAILED DESCRIPTION

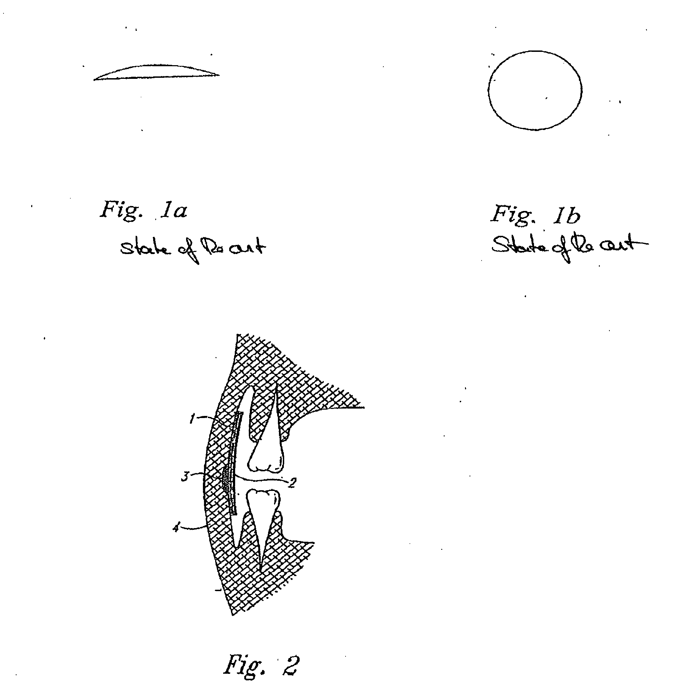

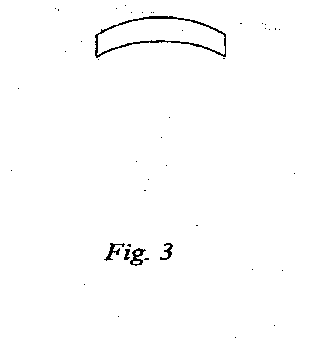

Figure 1 shows an adhesive oral patch that completely dissolves (more precisely, erodes as the molecules become hydrated). In the mouth, it has a feel and texture like hard gummy candies. It is made with slowly dissolving hydrocolloids so that that it typically lasts in the mouth for at least one to six hours. The patch can be formed in the shape of a tablet or a lozenge or a wafer or any other desired shape. A preferred shape for adhering to a tooth or braces that caused a cut that has become or is likely to become an ulcer is a dimpled dome - that is, convex on one side and concave on the other side. An example is shown in cross-section in Figure 3. The dimple may be a slight concavity. Nine millimeters diameter is a preferred size for such a dimpled dome made by pressing powders, with 0.5 to 1.5 millimeters for the depth of the dimple. For adhering to a bracket of orthodontic braces, such a dimple will allow greater contact with the bracket and wires for better adhesion. For adhering to a tooth, the concave dimple will allow the patch to adhere to a convex tooth surface at the periphery of the patch with multiple points of contact rather than with essentially a single point of contact near the center of a flat or convex patch surface.

A detailed description of a deposited patch and how to make it are disclosed by the same inventor in US patent application serial number 10/287,843 filed November 5, 2002 and published by the US Patent Office. A detailed description of a pressed powders patch made with mucoadhesive hydrocolloids pressed in two layers, one quite adhesive, entitled "Xylitol troches and methods of use" is disclosed by the same inventor in US patent application serial number 60/879846 filed January 11, 2007.

To cause the patch to dissolve (erode) very slowly in saliva, a binder that dissolves slowly in saliva is incorporated. Binders that have been tested and found to work well include gelatin, carrageenan (preferably kappa), xanthan gum, konjac gum, agar, gum arabic, and pectin. Other gums similar to those listed, such as locust bean gum which has properties similar to konjac gum, and guar gum should also work.

In addition to causing the patch to erode very slowly in the mouth, the binder also moderates any strong flavors by spreading out over a long period of time the release of that flavor. Consequently, sweeteners and other products to mask strong flavors are not required, although some users prefer a small amount of sweetener and some also prefer the addition of other flavors.

A method of manufacturing the patches of Figure 1 is to use gum drop manufacturing equipment, squirting a hydrated mixture heated above the gel melting temperature through nozzles onto a sheet of plastic or mold, allowing the patches to cool and gel, and drying the patches. The patches are preferably dried until the water activity level is lower than 0.8 so that the patches will not grow mold or other organisms. The patches are packaged with a hermetic seal to prevent absorption of water moisture from air. The resulting patches are at least 5 mm in each of at least two dimensions, preferably 8-18 mm.

The mixture may be deposited as an array of hot, viscous drops onto a sheet of high temperature plastic or coated paper. The drops are allowed to cool and then the sheets of plastic or coated paper with the drops on them are dried in a drying room. The product is sold still adhered to the plastic or paper and the user pulls it off the plastic or paper.

Figure 2 shows a bi-layer oral patch comprising a permeable layer **1** and a non-permeable smooth outer layer **2**. The oral patch is covering a canker sore **3** in a human cheek **4**. The outer layer **2** is preferably smooth to minimize dislodging of the patch. Medication is held in the permeable layer **1** either by using a high viscosity liquid medication that slowly oozes out of the layer or by binding the medication to the layer with slowly dissolving binders such as any of the gums described above, including gelatin. A preferred size for the patch is 18 millimeters, and one or both layers of the patch may include a red pigment to color it like the inside of the mouth.

Alternatively, any of the other oral patches known in the art may be used, such as patches made by heat a thermo gel mixture, extruding a flat sheet, and die cutting.

For an oral patch made by a tablet pressing process, the preferred size is about 100 to 150 milligrams for total tablet weight.

Glycyrrhetinic acid (GTA) is a mer component of glycyrrhizic acid, which is the negative part of the salt glycyrrhizin, which is a major ingredient in simple water extract of licorice root. When dissolved in water, the glycyrrhizic acid becomes bioavailable from the glycyrrhizin. Aided by the enzyme glucuronidase which is in all body fluids including saliva, this component hydrolyzes to release the glycyrrhetinic acid which causes undesirable side effects when taken in too large a quantity. However, in moderate quantities, the anti-inflammatory effect of glycyrrhetinic acid is desirable for reducing pain and speeding healing of ulcers because the quantities required are far below the side effect threshold, especially when a water soluble form of GTA (SGE) is used so that the GTA leaches well out of the patch and passes easily into the epithelium.

The preferred patch formulation is made by combining the GTA extract with collagen and with binder ingredients. Collagen, which is the organic molecule that makes up skin and the lining of the mouth (a form of skin), tends to adhere very well to itself, making it glutinous, and therefore adheres very well to the lining of the mouth. An effective and cost effective form of collagen is food grade gelatin which is made from animal skins. As the collagen molecules slough off the patch while it slowly dissolves (erodes), they tend to adhere to the nearby mouth lining, forming a film. This film significantly reduces the sensitivity of the ulcer, both to touch and to chemical irritants.

Testing shows that, if the binders are xanthan gum, konjac gum, and cellulose fiber, effective dry weight formulations have between 1% and 10% GTA, between 20% and 99% food grade gelatin, between 0% and 75% other binders.

Presented below are conclusions from testing on 49 subjects of the adherent, soluble oral patches with about 7-9% GTA:
**Pain relief:** Using a patch for 10-15 minutes before a meal reduces pain of the canker sore, and, if used up to commencement of a meal, the pain relief lasts through a typical meal. There is no numbing effect on surrounding tissues.
**Catching it early:** If the user catches the canker sore early, shorter treatment is required. The sore will often start in a small cut. Some users report that if they apply one patch to a cut for 1-4 hours before there is any sensation of a canker sore, then they will not get a canker sore from the cut. Other times, the sore starts with a feeling that the mucous layer is becoming too thin in a spot before it becomes painful. Some users report that if they apply one patch to that spot, no canker sore develops. Users report that if they begin applying the patch when the canker sore is very small and barely painful, the patches control the pain to the extent that there is no significant pain and healing is accelerated.
**Treatment of the tongue:** For treatment of the tongue, most users stick a patch (which releases extract on both sides) to the closest tooth. This works particularly well at night.
**Braces:** Users with braces apply the patch to the braces opposite the canker sore so that the patch is touching the canker sore most of the time and is stuck to the teeth and braces. As it softens, the patch settles into the braces. It will completely dissolve out of the braces in 3 - 9 hours. All this time it supplies GTA to the sore.

While particular embodiments of the invention have been described above the scope of the invention should not be limited by the above descriptions but rather limited only by the following claims.

## Claims

1. A dimpled, erodible, oral adhering troche for adhering to teeth or orthodontic braces, comprising:
a. an oral adhering troche of material that erodes in the human mouth, having a convex surface of at least 5 millimeters in at least two dimensions and a concave surface opposite the convex surface;
b. the concave surface comprising an adhesive that adheres to teeth or orthodontic braces

2. The troche of claim 1 being round and having a diameter of about 9 millimeters.

3. The troche of claim 1 wherein the troche releases glycyrrhetinic acid.

4. The troche of claim 1 wherein the troche releases benzocaine.

5. The troche of claim 1 wherein the troche releases collagen.

6. The troche of claim 1 wherein the concave surface has a depth of at least 0.5 millimeters.

7. The troche of claim 1 wherein the adhesive comprises acacia gum.

8. The troche of claim 1 wherein the adhesive comprises collagen.

9. The troche of any of the previous claims for the use in the treatment of mouth ulcer comprising:
the concave surface of the troche to be placed against a tooth or orthodontic brace with a patient in need thereof;
and allowing the troche to adhere to the tooth or the orthodontic braces, to remain in place, and to erode, thereby releasing the ingredient selected from the group comprising glycyrrhetinic acid, benzocaine and collagen.

## Patentansprüche

1. Eine mit Vertiefung versehene, erodierbare, oral-haftende Pastille zum Anhaften an Zähnen oder Zahnspangen, umfassend:
a. eine oral-haftende Pastille aus Material, das im menschlichen Mund erodiert, mit einer konvexen Oberfläche von wenigstens 5 Millimetern in wenigstens zwei Dimensionen und einer der konvexen Oberfläche gegenüberliegenden konkaven Oberfläche;
b. wobei die konkave Oberfläche einen Haftstoff umfasst, der an Zähnen oder Zahnspangen anhaftet.

2. Pastille gemäß Anspruch 1, die rund ist und einen Durchmesser von ungefähr 9 Millimetern hat

3. Pastille gemäß Anspruch 1, wobei die Pastille Glycyrrhetinsäure freisetzt.

4. Pastille gemäß Anspruch 1, wobei die Pastille Benzocain freisetzt.

5. Pastille gemäß Anspruch 1, wobei die Pastille Collagen freisetzt.

6. Pastille gemäß Anspruch 1, wobei die konkave Oberfläche eine Tiefe von wenigstens 0,5 Millimetern hat.

7. Pastille gemäß Anspruch 1, wobei der Haftstoff Gummiarabikum umfasst.

8. Pastille gemäß Anspruch 1, wobei der Haftstoff Collagen umfasst.

9. Pastille gemäß einem der vorhergehenden Ansprüche zur Verwendung bei der Behandlung von Mundgeschwüren, umfassend:
die konkave Oberfläche der Pastille zum Anlegen an einen Zahn oder eine Zahnspange bei einem Patienten mit Bedarf daran; und
wobei ein Anhaften der Pastille an dem Zahn oder der Zahnspange, Verbleib derselben an dieser Stelle und Erodieren derselben zugelassen ist, wodurch der Inhaltsstoff freigesetzt wird, der aus der Gruppe ausgewählt ist, die Glycyrrhetinsäure, Benzocain und Collagen umfasst.

## Revendications

1. Pastille orale adhésive avec fossette en vue de l'adhésion à des dents ou des bandes orthodontiq ues, comprenant ;
a. une pastille orale adhésive de matière qui se dissout dans la bouche humaine, possédant une surface convexe d'au moins 5 millimètres dans au moins deux dimensions et une surface concave opposée à la surface convexe;
b. la surface concave comprenant un adhésif qui adhère aux dents ou aux bandes orthodontiques.

2. Pastille selon la revendication 1, étant ronde et possédant un diamètre d'environ 9 millimètres.

3. Pastille selon la revendication 1, dans laquelle la pastille relâche de l'acide glycyrrhétinique.

4. Pastille selon la revendication 1, dans laquelle la pastille relâche de la benzocaïne.

5. Pastille selon la revendication 1, dans laquelle la pastille relâche du collagène.

6. Pastille selon la revendication 1, dans laquelle la surface concave possède une profondeur d'au moins 0,5 millimètres.

7. Pastille selon la revendication 1, dans laquelle l'adhésif comprend de la gomme d'acacia.

8. Pastille selon la revendication 1, dans laquelle l'adhésif comprend du collagène.

9. Pastille selon une quelconque des revendications précédentes en vue de l'utilisation dans le traitement de l'ulcère buccale comprenant:
la pastille à placer avec la surface concave de la pastille contre une dent ou une bande orthodontique pour un patient nécessiteux de celle-ci; et
l'autorisation de la pastille à adhérer à la dent ou aux bandes orthodontiques, à rester en place et à se dissoudre, relâchant alors l'ingrédient sélectionné à partir du groupe comprenant de l'acide glycyrrhétinique, de la benzocaïne et du collagène.
